# FASCICULE DE BREVET EUROPEEN

(11) **EP 2 964 622 B1**
(45) Date de publication et mention de la délivrance du brevet: **19.04.2017**
(21) Numéro de dépôt: 14711833.5
(22) Date de dépôt: 03.03.2014
(51) Int. Cl.: C07D 307/85, A61K 31/343, A61P 29/00, A61P 35/00

(54) **NOUVEAU SEL DE L'ABEXINOSTAT, FORME CRISTALLINE ASSOCIEE, LEUR PROCEDE DE PREPARATION ET LES COMPOSITIONS PHARMACEUTIQUES QUI LES CONTIENNENT**
NEUARTIGES ABEXINOSTATSALZ, ZUGEHÖRIGE KRISTALLINE FORM, HERSTELLUNGSVERFAHREN DAFÜR UND PHARMAZEUTISCHE ZUSAMMENSETZUNGEN DAMIT
NOVEL ABEXINOSTAT SALT, ASSOCIATED CRYSTALLINE FORM, PREPARATION METHOD THEREOF AND THE PHARMACEUTICAL COMPOSITIONS CONTAINING SAME

(30) Priorité: 04.03.2013 FR 1351898; 04.03.2013 US 201361772191 P
(43) Date de publication de la demande: 13.01.2016
(73) Titulaire: Pharmacyclics LLC, Sunnyvale, CA 94085 (US)
(72) Inventeur: PIMONT-GARRO, Anne, F-78000 Versailles (FR); LETELLIER, Philippe, F-45000 Orléans (FR)
(74) Mandataire: HGF Limited
(86) Numéro de dépôt international: PCT/FR2014/050455
(87) Numéro de publication internationale: WO 2014/135776

(56) Documents cités:
- WO-A1-2010/123507
- WO-A2-2004/092115

## Description

La présente invention concerne le tosylate de *N*-hydroxy-4-{2-[3-(*N,N-*diméthylaminométhyl)benzofuran-2-ylcarbonylamino]éthoxy}benzamide, ou l'un de ses solvates.

Alternativement, l'objet de l'invention concerne un sel de tosylate de l'abexinostat de formule (I) :

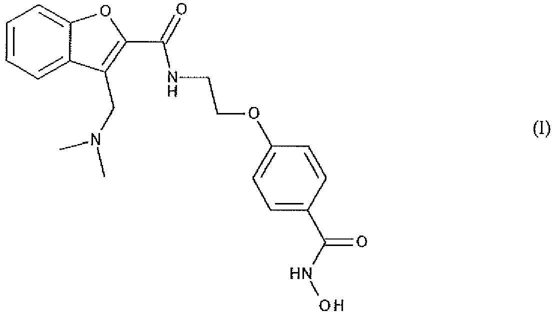

Plus particulièrement, le sel de formule (II) est visé :

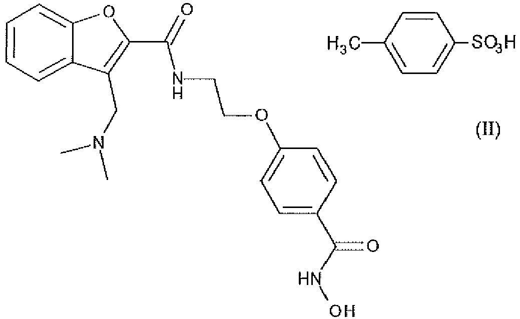

La présente invention concerne également la forme cristalline I du tosylate de *N*-hydroxy-4-{2-[3-(*N*,*N*-diméthylaminométhyl)benzofuran-2-ylcarbonylamino]éthoxy}benzamide, son procédé de préparation ainsi que les compositions pharmaceutiques qui la contiennent.

Le *N*-hydroxy-4-{2-[3-(*N,N*-diméthylaminométhyl)benzofuran-2-ylcarbonylamino] éthoxy}benzamide, aussi appelé abexinostat, est un inhibiteur des histones-déacétylases (HDAC) décrit dans la demande de brevet WO2004/092115. Il permet d'inhiber la croissance cellulaire et induit l'apoptose dans des cellules tumorales cultivées *in vitro,* et inhibe la croissance tumorale *in vivo* dans des modèles de xénogreffes (Buggy et al Mol. Cancer Ther 2006 5(5) 1309). Compte-tenu de son profil pharmacologique, l'abexinostat est destiné à être utilisé dans le traitement du cancer.

Du point de vue industriel, il est primordial de pouvoir synthétiser le composé avec une excellente pureté, et notamment sous une forme parfaitement reproductible, présentant des caractéristiques intéressantes de dissolution, de filtration, de séchage, de facilité de formulation et de stabilité permettant son stockage prolongé sans conditions particulières de température, de lumière, d'humidité ou de taux d'oxygène.

La demande de brevet WO2004/092115 décrit deux voies d'accès différentes pour obtenir l'abexinostat. Dans les deux cas, l'acide 3-méthyl-benzofuran-2-carboxylique est utilisé comme produit de départ, mais la fonctionnalisation de ce noyau central par le groupement diméthylaminométhyl en position 3 est réalisée à des étapes différentes du procédé de synthèse, en l'occurrence avant ou après le couplage du dérivé de l'acide benzofuran-2-carboxylique avec le 4-(2-aminoéthoxy)benzoate de méthyle. L'obtention du chlorhydrate d'abexinostat est spécifiquement décrite dans la demande WO2004/092115. Cependant, l'utilisation de ce sel à une échelle industrielle est délicate du fait de ses propriétés hygroscopiques.

La présente invention décrit un procédé d'obtention du tosylate d'abexinostat (4-méthylbenzènesulfonate d'abexinostat) sous une forme cristalline bien définie, parfaitement reproductible, et présentant une très bonne stabilité compatible avec les contraintes industrielles de préparation (de séchage en particulier) et de conservation des compositions pharmaceutiques.

La forme cristalline I du tosylate d'abexinostat est caractérisée par un diagramme de diffraction X sur poudre présentant les raies de diffraction suivantes (angle de Bragg 2 thêta, exprimé en degrés ±0,2°) : 6,50 ; 9,94 ; 11,35 ; 12,33 ; 14,08 ; 18,95 ; 21,08 ; 27,05. Plus particulièrement encore, la forme cristalline I du tosylate d'abexinostat est caractérisée par les raies de diffraction suivantes: 6,50; 9,94; 11,35 ; 12,33 ; 14,08; 18,95 ; 19,61 ; 19,96 ; 21,08 ; 22,82 ; 23,61 ; 27,05.

Plus spécifiquement, la forme cristalline I du tosylate d'abexinostat est caractérisée par le diagramme de diffraction X sur poudre ci-dessous, mesuré à l'aide d'un diffractomètre PANalytical X'Pert Pro MPD avec un détecteur X'Celerator, et exprimé en termes de position de raie (angle de Bragg 2 thêta, exprimé en degrés ±0,2°) et de distance inter-réticulaire d (exprimée en Å) :

| **Raie n°** | **Angle 2-thêta (degrés)** | **Distance inter-réticulaire (Å)** |
|---|---|---|
| 1 | 6,50 | 13,581 |
| 2 | 9,94 | 8,894 |
| 3 | 11,35 | 7,789 |
| 4 | 12,33 | 7,173 |
| 5 | 14,08 | 6,285 |
| 6 | 18,95 | 4,683 |
| 7 | 19,61 | 4,526 |
| 8 | 19,96 | 4,449 |
| 9 | 21,08 | 4,215 |
| 10 | 22,82 | 3,897 |
| 11 | 23,61 | 3,768 |
| 12 | 27,05 | 3,296 |

En outre, la forme cristalline I du tosylate d'abexinostat a été caractérisée par spectroscopie Raman. Des pics significatifs ont été observés aux positions suivantes : 940 cm⁻¹, 1088 cm⁻¹, 1132 cm⁻¹, 1242 cm⁻¹, 1360 cm⁻¹, 1608 cm⁻¹.

Alternativement, la forme cristalline I du tosylate d'abexinostat peut être caractérisée par le diagramme de diffraction X sur poudre comportant les 12 raies significatives présentées précédemment, ainsi que par un spectre Raman présentant un pic significatif à la position 1608 cm⁻¹.

Enfin, la forme cristalline I du tosylate d'abexinostat a également été caractérisée par spectroscopie RMN à l'état solide. Des pics significatifs ont été observés à 121,2 ppm, 122,1 ppm, 123,5 ppm, 126,0 ppm, 126,8 ppm, 128,2 ppm, 128,9 ppm, 143,4 ppm, 144,6 ppm, 153,8 ppm, 159 ppm, 161,2 ppm et 162,1 ppm.
Plus précisément, les spectres ¹³C CP/MAS (Cross Polarization Magic Angle Spinning) présentent les pics suivants (exprimés en ppm ± 0,2 ppm) :

| **Pic n°** | **Déplacement chimique (ppm)** | **Pic n°** | **Déplacement chimique (ppm)** |
|---|---|---|---|
| 1 | 162,1 | 10 | 126,0 |
| 2 | 161,2 | 11 | 123,5 |
| 3 | 159,0 | 12 | 122,1 |
| 4 | 153,8 | 13 | 121,3 |
| 5 | 144,6 | 14 | 65,9 |
| 6 | 143,4 | 15 | 50,6 |
| 7 | 128,9 | 16 | 46,9 |
| 8 | 128,2 | 17 | 45,0 |
| 9 | 126,8 | 18 | 21,9 |

L'invention s'étend également au procédé de préparation de la forme cristalline I du tosylate d'abexinostat, caractérisé en ce que l'abexinostat est cristallisé dans un milieu polaire en présence d'acide *para*-toluènesulfonique. De manière préférentielle, le milieu polaire est constitué d'un ou plusieurs solvants choisis parmi l'eau, les alcools, les cétones et les esters, étant entendu que :
- par "alcools", on entend les alcools C₁-C₆ tels que le méthanol, l'éthanol, le propanol, l'isopropanol, le butanol, l'isobutanol, le pentanol, le 2-pentanol, le 3-pentanol, l'isopentanol, l'hexanol,
- par "cétones", on entend les cétones C₃-C₆ telles que l'acétone, la méthyléthyl cétone, la 2-pentanone, la 3-pentanone, la 3-méthyl-2-butanone, la 2-hexanone, la 3-hexanone, l'éthylisopropylcétone, la méthylisopropylcétone, la 2,2-diméthyl-3-butanone,
- par "esters", on entend les esters C₃-C₈ tels que le formate d'éthyle, le formate d'isopropyle, l'acétate d'éthyle, l'acétate de propyle, l'acétate d'isopropyle, l'acétate de butyle, l'acétate d'isobutyle, l'acétate de *tert*-butyle, l'acétate de pentyle, l'acétate d'isopentyle, l'acétate d'hexyle.

Les alcools préférés sont l'éthanol et l'isopropanol. Parmi les solvants préférés, on retiendra également l'acétone et la méthyléthylcétone pour les cétones, et l'acétate d'éthyle pour les esters.

Alternativement, le milieu polaire est un mélange binaire dont l'un des constituants est l'eau. Plus préférentiellement encore, le milieu polaire est un mélange binaire choisi parmi : acétone/eau, éthanol/eau, isopropanol/eau et méthyléthylcétone/eau.

Dans le procédé de cristallisation selon l'invention, on peut utiliser l'abexinostat (base libre) obtenu par n'importe quel procédé.

L'invention s'étend également à un autre procédé de préparation de la forme cristalline I du tosylate d'abexinostat, dans lequel la cristallisation est amorcée par une très petite quantité de la forme cristalline 1 du tosylate d'abexinostat.
Dans ce second procédé de cristallisation selon l'invention, on peut aussi utiliser l'abexinostat (base libre) obtenu par n'importe quel procédé.

L'obtention de la forme cristalline 1 du tosylate d'abexinostat a pour avantage de permettre la préparation de formulations pharmaceutiques ayant une composition constante et reproductible, présentant de bonnes caractéristiques de dissolution et de stabilité, ce qui est particulièrement avantageux lorsque les formulations sont destinées à l'administration orale. Plus précisément, l'utilisation de la forme cristalline I du tosylate d'abexinostat est particulièrement intéressante sur le plan industriel compte-tenu de sa faible hygroscopicité.

La forme cristalline I du tosylate d'abexinostat est destinée au traitement du cancer, et plus particulièrement au traitement d'un carcinome, d'une tumeur, d'un néoplasme, d'un lymphome, d'un mélanome, d'un gliome, d'un sarcome, ou d'un blastome.

L'invention s'étend aussi aux compositions pharmaceutiques renfermant comme principe actif un sel de tosylate de l'abexinostat, et plus particulièrement encore la forme cristalline I du tosylate d'abexinostat, avec un ou plusieurs excipients inertes, non toxiques et appropriés. Parmi les compositions pharmaceutiques selon l'invention, on pourra citer plus particulièrement celles qui conviennent pour l'administration orale, parentérale (intraveineuse ou sous-cutanée), nasale, les comprimés simples ou dragéifiés, les granulés, les comprimés sublinguaux, les gélules, les tablettes, les suppositoires, les crèmes, les pommades, les gels dermiques, les préparations injectables, les suspensions buvables et les pâtes à mâcher.

Les compositions pharmaceutiques administrées par voie orale sont préférées.

La posologie utile varie selon le sexe, l'âge et le poids du patient, la voie d'administration, la nature du cancer et des traitements éventuellement associés et la posologie utile s'échelonne entre 20 mg et 480 mg de *N*-hydroxy-4-{2-[3-(*N,N-*diméthylaminométhyl)benzo-furan-2-ylcarbonylamino]éthoxy}benzamide exprimé en base libre par jour.

Les exemples ci-dessous illustrent l'invention, mais ne la limitent en aucune façon.

### Exemple 1: Procédé d'obtention de la forme cristalline I du tosylate d'abexinostat

1,66 kg d'abexinostat (base libre) sont placés dans 9,48 kg d'un mélange d'isopropanol/eau (50/50 masse/masse) à température ambiante. L'acide *para*-toluènesulfonique monohydraté (0,83 kg) est ajouté dans 2,36 kg d'eau à température ambiante. Le milieu est ensuite chauffé à 75°C pendant 30 minutes avant d'être refroidi à 0°C. Lorsque la cristallisation est totale, la suspension est filtrée à 20°C. Après séchage, la forme cristalline I du tosylate d'abexinostat est obtenue avec un rendement d'environ 85% et une pureté supérieure à 99%. Le solide a été caractérisé par le diagramme de diffraction X sur poudre, le spectre Raman et le spectre RMN détaillés aux Exemples 3-6 suivants.

### Exemple 2: Procédé d'obtention de la forme cristalline I du tosylate d'abexinostat (ensemencement)

33,9 kg d'abexinostat (base libre) sont placés dans 170 kg d'un mélange d'isopropanol/eau (45,6/54,4 masse/masse) à température ambiante. Une solution constituée d'acide *para-*toluènesulfonique monohydraté (17,06 kg) dans l'eau (24,1 kg) est ajoutée. Le milieu est ensuite chauffé à 70-75°C, refroidi et amorcé avec 1,935 kg de forme cristalline I de tosylate d'abexinostat. La suspension est ensuite filtrée à 20°C. Après séchage, la forme cristalline I du tosylate d'abexinostat est obtenue avec un rendement d'environ 86% et une pureté supérieure à 99%. Le solide a été caractérisé par le diagramme de diffraction X sur poudre, le spectre Raman et le spectre RMN détaillés aux Exemples 3-6 suivants.

### Exemple 3: Forme cristalline I du tosylate d'abexinostat (diagramme de diffraction X sur poudre)

L'enregistrement des données a été effectué sur un diffractomètre PANalytical X'Pert Pro MPD avec un détecteur X'Celerator dans les conditions suivantes :
- Tension 45 kV, intensité 40 mA,
- Montage thêta/thêta,
- Anode : cuivre,
- Longueur d'onde K alpha-1 : 1,54060 Å,
- Longueur d'onde K alpha-2 : 1,54443 Å,
- Rapport K alpha-2/ K alpha-1 : 0,5
- Mode de mesure : continu de 3° à 55° (angle de Bragg 2 thêta) avec une incrémentation de 0,017°,
- Temps de mesure par pas : 35,53 s.

Le diagramme de diffraction X sur poudre de la forme I du tosylate d'abexinostat obtenu selon le procédé de l'Exemple 1 ou 2 est exprimé en terme de position de raie (angle de Bragg 2 thêta, exprimé en degrés ±0,2°), de distance inter-réticulaire d (exprimée en Å) et d'intensité relative (exprimée en pourcentage par rapport à la raie la plus intense). Les raies significatives ont été rassemblées dans le tableau suivant :

| **Raie n°** | **Angle 2-thêta (degrés)** | **Distance inter-réticulaire (Å)** | **Intensité relative (%)** |
|---|---|---|---|
| 1 | 6,50 | 13,581 | 75,6 |
| 2 | 9,94 | 8,894 | 58,4 |
| 3 | 11,35 | 7,789 | 19,1 |
| 4 | 12,33 | 7,173 | 23,7 |
| 5 | 14,08 | 6,285 | 33,1 |
| 6 | 18,95 | 4,683 | 100 |
| 7 | 19,61 | 4,526 | 53,9 |
| 8 | 19,96 | 4,449 | 50,9 |
| 9 | 21,08 | 4,215 | 93,5 |
| 10 | 22,82 | 3,897 | 28,5 |
| 11 | 23,61 | 3,768 | 32,6 |
| 12 | 27,05 | 3,296 | 16,0 |

### Exemple 4: Forme cristalline I du tosylate d'abexinostat (maille cristalline)

Une solution saturée de tosylate d'abexinostat dans le 2,2,2-trifluoroéthanol est préparée par agitation d'une suspension pendant 24 heures à température ambiante, suivie d'une filtration. On verse ensuite 1 mL de cette solution saturée dans un vial HPLC de 1,8 mL auquel on ajoute 0,25 mL d'eau. La solution est maintenue à température ambiante pendant 75 minutes. Après centrifugation puis séchage, le solide est isolé pour analyse. Parmi les cristaux obtenus, un cristal de qualité suffisante est prélevé pour analyse par diffraction X sur monocristal.

La structure cristalline a été déterminée sur le monocristal précédent à l'aide d'un diffractomètre Bruker Kappa CCD équipé d'un générateur FR590 à anticathode molybdène ((λMoKα1 = 0,7093 Å) avec un domaine angulaire compris entre 2° et 27,5° en θ. Les paramètres suivants ont été établis :
- maille cristalline triclinique,
- paramètres de maille : a = 10,467 Å, b = 14,631 Â, c = 20,159 Å, α = 73,971°, β = 79,040°, γ = 72,683°
- groupe d'espace : P -1
- nombre de molécules dans la maille : 4
- volume de la maille : Vₘₐᵢₗₗₑ = 2813,0 Å³
- densité : d = 1,345 g/_{cm}³.

### Exemple 5: Forme cristalline I du tosylate d'abexinostat (spectre Raman)

La forme I du tosylate d'abexinostat a été caractérisée par spectroscopie Raman. Les spectres ont été enregistrés en mode réflexion diffuse (Raman Station 400, PerkinElmer) avec un laser à 785 nm. Le signal a été enregistré par un détecteur CCD. Le décalage en longueur d'onde dépend de la matière et lui est caractéristique, ce qui permet une analyse de la composition chimique et de l'agencement moléculaire de l'échantillon étudié. Les spectres ont été acquis avec une puissance maximale (100% de la capacité du laser), une taille de spot de 100 µm, vingt expositions de 2 secondes et une résolution spectrale de 2 cm⁻¹. La gamme spectrale explorée s'échelonne entre 0 et 3278 cm⁻¹.

Des pics significatifs ont été observés aux positions suivantes : 940 cm⁻¹, 1088 cm⁻¹, 1132 cm⁻¹, 1242 cm⁻¹, 1360 cm⁻¹, 1608 cm⁻¹.

### Exemple 6: Forme cristalline I du tosylate d'abexinostat (spectre RMN Solide)

La forme I du tosylate d'abexinostat a également été caractérisée par spectroscopie RMN à l'état solide. Les spectres ¹³C RMN ont été enregistrés à température ambiante à l'aide d'un spectromètre Bruker SB Avance avec une sonde de type 4 mm CP/MAS SB VTN dans les conditions suivantes :
- Fréquence : 125,76 MHz,
- Largeur spectrale : 40 kHz,
- Vitesse de rotation de l'échantillon à l'angle magique : 10 kHz,
- Séquence d'impulsion : CP (Cross Polarization) avec découplage SPINAL64 (puissance de découplage de 80 kHz),
- Délai de répétitions : 10 s,
- Temps d'acquisition : 35 ms,
- Temps de contact : 4 ms
- Nombre de scans : 4096.

Une fonction d'apodisation (« 5 Hz line broadening ») est appliquée au signal recueilli avant la transformée de Fourier. Les spectres ainsi obtenus ont été référencés par rapport à un échantillon d'adamantane (le pic de plus haute fréquence de l'adamantane a pour déplacement chimique 38,48 ppm).

Les pics observés ont été rassemblés dans le tableau suivant (exprimés en ppm ± 0,2 ppm) :

| **Pic n°** | **Déplacement chimique (ppm)** | **Pic n°** | **Déplacement chimique (ppm)** |
|---|---|---|---|
| 1 | 162,1 | 10 | 126,0 |
| 2 | 161,2 | 11 | 123,5 |
| 3 | 159,0 | 12 | 122,1 |
| 4 | 153,8 | 13 | 121,3 |
| 5 | 144,6 | 14 | 65,9 |
| 6 | 143,4 | 15 | 50,6 |
| 7 | 128,9 | 16 | 46,9 |
| 8 | 128,2 | 17 | 45,0 |
| 9 | 126,8 | 18 | 21,9 |

### Exemple 7: Composition pharmaceutique

Formule de préparation pour 1000 comprimés dosés à 100 mg d'abexinostat (exprimé en équivalent base) :

| | |
|---|---|
| Tosylate d'abexinostat | 143,4 g |
| Lactose monohydrate | 213,1 g |
| Stéarate de Magnésium | 2,5 g |
| Amidon de maïs | 75 g |
| Maltodextrine | 50 g |
| Silice colloïdale anhydre | 1 g |
| Carboxymethyl cellulose sodique | 15 g |

### Exemple 8: Hygroscopie

L'hygroscopicité de la forme I du tosylate d'abexinostat a été évaluée par gravimétrie d'adsorption de vapeur d'eau (DVS - *Dynamic Vapor Sorption*). Un échantillon de 5 à 10 mg de la substance médicamenteuse, pesé avec précision, a été disposé dans une cuve d'échantillon DVS fonctionnant à 25°C sous humidité contrôlée. La variation de masse a été enregistrée au cours d'un séchage sous 0 pourcent HR (humidité relative) et durant deux cycles subséquents d'augmentation et de diminution linéaires de l'humidité relative dans la plage de 0-90 pourcent HR à une vitesse de 10 pourcent par heure. L'humidité relative a été maintenue constante lorsqu'elle a atteint soit 0 soit 90 pourcent HR, jusqu'à ce que la variation de masse soit inférieure à 0,002 pourcent par minute, avec une limite en temps de 15h.

Une augmentation en poids inférieure à 0,5% a été détectée par analyse DVS après exposition d'un échantillon à des humidités relatives comprises entre 0% et 90% à 25°C.

## Revendications

1. Tosylate de *N*-hydroxy-4-{2-[3-(*N,N*-diméthylaminométhyl)benzofuran-2-ylcarbonylamino]éthoxy}benzamide, ou l'un de ses solvates.

2. Sel selon la revendication 1 de formule (II) :

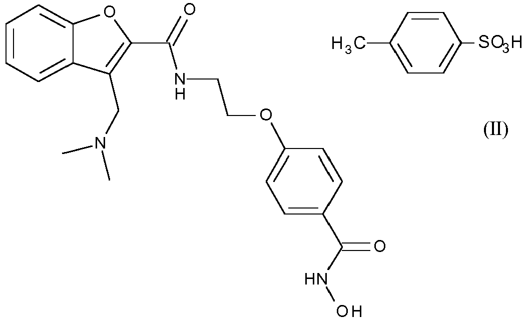

3. Forme cristalline I du tosylate d'abexinostat selon l'une des revendications 1 ou 2 **caractérisée en ce qu'**elle a un diagramme de diffraction X sur poudre présentant les raies de diffraction suivantes (angle de Bragg 2 thêta, exprimé en degrés ±0,2°) : 6,50 ; 9,94 ; 11,35 ; 12,33 ; 14,08 ; 18,95 ; 21,08 ; 27,05.

4. Forme cristalline I du tosylate d'abexinostat selon la revendication 3 **caractérisée en ce qu'**elle a un diagramme de diffraction X sur poudre présentant les raies de diffraction suivantes (angle de Bragg 2 thêta, exprimé en degrés ±0,2°) : 6,50 ; 9,94 ; 11,35 ; 12,33 ; 14,08 ; 18,95 ; 19,61 ; 19,96 ; 21,08 ; 22,82 ; 23,61 ; 27,05.

5. Forme cristalline I du tosylate d'abexinostat selon la revendication 3 ou 4 **caractérisée en ce qu'**elle a le diagramme de diffraction X sur poudre suivant, mesuré sur un diffractomètre PANalytical X'Pert Pro MPD avec un détecteur X'Celerator, et exprimé en termes de position de raie (angle de Bragg 2 thêta, exprimé en degrés ±0,2°), et de distance inter-réticulaire d (exprimée en Å) :
| **Raie n°** | **Angle 2-thêta (degrés)** | **Distance inter-réticulaire (Å)** |
|---|---|---|
| 1 | 6,50 | 13,581 |
| 2 | 9,94 | 8,894 |
| 3 | 11,35 | 7,789 |
| 4 | 12,33 | 7,173 |
| 5 | 14,08 | 6,285 |
| 6 | 18,95 | 4,683 |
| 7 | 19,61 | 4,526 |
| 8 | 19,96 | 4,449 |
| 9 | 21,08 | 4,215 |
| 10 | 22,82 | 3,897 |
| 11 | 23,61 | 3,768 |
| 12 | 27,05 | 3,296 |

6. Forme cristalline I du tosylate d'abexinostat selon l'une des revendications 3 à 5 **caractérisée en ce qu'**elle a un spectre Raman présentant un pic significatif à la position 1608 cm⁻¹.

7. Forme cristalline I du tosylate d'abexinostat selon l'une des revendications 1 à 6 **caractérisée en ce qu'**elle a un spectre Raman présentant des pics significatifs aux positions 940 cm⁻¹, 1088 cm⁻¹, 1132 cm⁻¹, 1242 cm⁻¹, 1360 cm⁻¹, 1608 cm⁻¹.

8. Forme cristalline I du tosylate d'abexinostat selon l'une des revendications 1 ou 2 **caractérisée en ce qu'**elle a un spectre RMN à l'état solide ¹³C CP/MAS présentant les pics suivants (exprimés en ppm ± 0,2 ppm) : 121,2 ppm, 122,1 ppm, 123,5 ppm, 126,0 ppm, 126,8 ppm, 128,2 ppm, 128,9 ppm, 143,4 ppm, 144,6 ppm, 153,8 ppm, 159 ppm, 161,2 ppm et 162,1 ppm.

9. Forme cristalline I du tosylate d'abexinostat selon la revendication 8 **caractérisée en ce qu'**elle a un spectre RMN à l'état solide ¹³C CP/MAS présentant les pics suivants (exprimés en ppm ±0,2 ppm) :
| **Pic n°** | **Déplacement chimique (ppm)** | **Pic n°** | **Déplacement chimique (ppm)** |
|---|---|---|---|
| 1 | 162,1 | 10 | 126,0 |
| 2 | 161,2 | 11 | 123,5 |
| 3 | 159,0 | 12 | 122,1 |
| 4 | 153,8 | 13 | 121,3 |
| 5 | 144,6 | 14 | 65,9 |
| 6 | 143,4 | 15 | 50,6 |
| 7 | 128,9 | 16 | 46,9 |
| 8 | 128,2 | 17 | 45,0 |
| 9 | 126,8 | 18 | 21,9 |

10. Composition pharmaceutique contenant comme principe actif le tosylate d'abexinostat selon l'une des revendications 1 ou 2 en association avec un ou plusieurs excipients pharmaceutiquement acceptables.

11. Composition pharmaceutique contenant comme principe actif la forme cristalline I du tosylate d'abexinostat selon l'une des revendications 3 à 9, en association avec un ou plusieurs excipients pharmaceutiquement acceptables.

12. Composition pharmaceutique selon la revendication 10 ou 11 pour son utilisation dans le traitement du cancer.

13. Composition pharmaceutique destinée à être utilisée selon l'une des revendications 10 à 12 dans laquelle le cancer est un carcinome, une tumeur, un néoplasme, un lymphome, un mélanome, un gliome, un sarcome ou un blastome.

14. Procédé de préparation de la forme cristalline I du tosylate d'abexinostat selon l'une des revendications 3 à 9, dans lequel l'abexinostat est cristallisé en présence d'acide *para-*toluènesulfonique dans un milieu polaire.

15. Procédé de préparation de la forme cristalline I du tosylate d'abexinostat selon la revendication 14, dans lequel le milieu polaire est constitué d'un ou plusieurs solvants choisis parmi l'eau, les alcools, les cétones et les esters.

16. Procédé de préparation de la forme cristalline I du tosylate d'abexinostat selon la revendication 15, dans lequel le milieu polaire est un mélange binaire dont l'un des constituants est l'eau.

17. Procédé de préparation de la forme cristalline I du tosylate d'abexinostat selon la revendication 16, dans lequel le milieu polaire est un mélange binaire choisi parmi : acétone/eau, éthanol/eau, isopropanol/eau et méthyléthylcétone/eau.

18. Procédé de préparation de la forme cristalline I du tosylate d'abexinostat selon l'une des revendications 14 à 17, dans lequel la cristallisation est amorcée par une très petite quantité de la forme cristalline I du tosylate d'abexinostat.

## Patentansprüche

1. Tosylat von *N*-hydroxy-4-{2-[3-(*N,N*-dimethylaminomethyl)benzofuran-2-ylcarbonylamino]ethoxy}benzamid oder ein Solvat davon.

2. Salz nach dem Anspruch 1 der Formel (II):

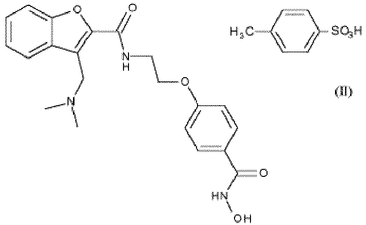

3. Kristallform I des Abexinostat Tosylats nach einem der Ansprüche 1 oder 2, **dadurch gekennzeichnet, dass** sie ein Pulver-Röntgenbeugungsdiagramm aufweist, das die folgenden Beugungslinien (Bragg-Winkel 2 Theta, ausgedrückt in Grad ±0,2°) aufweist: 6,50; 9,94; 11,35; 12,33; 14,08; 18,95; 21,08; 27,05.

4. Kristallform I des Abexinostat Tosylats nach Anspruch 3, **dadurch gekennzeichnet, dass** sie ein Pulver-Röntgenbeugungsdiagramm aufweist, das die folgenden Beugungslinien (Bragg-Winkel 2 Theta, ausgedrückt in Grad ±0,2°) aufweist: 6,50; 9,94; 11,35; 12,33; 14,08; 18,95; 19,61; 19,96; 21,08; 22,82; 23,61; 27,05.

5. Kristallform I des Abexinostat Tosylats nach Anspruch 3 oder 4, **dadurch gekennzeichnet, dass** sie das folgende Pulver-Röntgenbeugungsdiagramm, gemessen mit einem Diffraktometer PANalytical X'Pert Pro MPD mit einem Detektor X'Celerator und ausgedrückt als Linienposition (Bragg-Winkel 2 Theta, ausgedrückt in Grad ±0,2°) und Netzebenenabstand d (ausgedrückt in A) aufweist:
| Linie Nr. | Winkel 2 Theta (Grade) | Netzebenenabstand (Å) |
|---|---|---|
| 1 | 6,50 | 13,581 |
| 2 | 9,94 | 8,894 |
| 3 | 11,35 | 7,789 |
| 4 | 12,33 | 7,173 |
| 5 | 14,08 | 6,285 |
| 6 | 18,95 | 4,683 |
| 7 | 19,61 | 4,526 |
| 8 | 19,96 | 4,449 |
| 9 | 21,08 | 4,215 |
| 10 | 22,82 | 3,897 |
| 11 | 23,61 | 3,768 |
| 12 | 27,05 | 3,296 |

6. Kristallform I des Abexinostat Tosylats nach einem der Ansprüche 3 bis 5, **dadurch gekennzeichnet, dass** sie ein Raman-Spektrum mit einem signifikanten Peak in der Position 1608 cm⁻¹ aufweist.

7. Kristallform I des Abexinostat Tosylats nach einem der Ansprüche 1 bis 6, **dadurch gekennzeichnet, dass** sie ein Raman-Spektrum mit signifikanten Peaks in den Positionen 940 cm⁻¹, 1088 cm⁻¹, 1132 cm⁻¹, 1242 cm⁻¹, 1360 cm⁻¹, 1608 cm⁻¹ aufweist.

8. Kristallform I des Abexinostat Tosylats nach einem der Ansprüche 1 oder 2, **dadurch gekennzeichnet, dass** sie ein Festphasen-¹³C CP/MAS NMR-Spektrum mit den folgenden Peaks (ausgedrückt in ppm ± 0,2 ppm) aufweist: 121,2 ppm, 122,1 ppm, 123,5 ppm, 126,0 ppm, 126,8 ppm, 128,2 ppm, 128,9 ppm, 143,4 ppm, 144,6 ppm, 153,8 ppm, 159 ppm, 161,2 ppm und 162,1 ppm.

9. Kristallform I des Abexinostat Tosylats nach Anspruch 8, **dadurch gekennzeichnet, dass** sie ein Festphasen-¹³C CP/MAS NMR-Spektrum mit den folgenden Peaks (ausgedrückt in ppm ± 0,2 ppm) aufweist:
| Peak Nr. | Chemische Verschiebung (ppm) | Peak Nr. | Chemische Verschiebung (ppm) |
|---|---|---|---|
| 1 | 162,1 | 10 | 126,0 |
| 2 | 161,2 | 11 | 123,5 |
| 3 | 159,0 | 12 | 122,1 |
| 4 | 153,8 | 13 | 121,3 |
| 5 | 144,6 | 14 | 65,9 |
| 6 | 143,4 | 15 | 50,6 |
| 7 | 128,9 | 16 | 46,9 |
| 8 | 128,2 | 17 | 45,0 |
| 9 | 126,8 | 18 | 21,9 |

10. Pharmazeutische Zusammensetzung, umfassend als Wirkstoff das Abexinostat Tosylat nach einem der Ansprüche 1 oder 2 in Verbindung mit einem oder mehreren pharmazeutisch annehmbaren Exzipienten.

11. Pharmazeutische Zusammensetzung, umfassend als Wirkstoff die Kristallform I des Abexinostat Tosylats nach einem der Ansprüche 3 bis 9 in Verbindung mit einem oder mehreren pharmazeutisch annehmbaren Exzipienten.

12. Pharmazeutische Zusammensetzung nach Anspruch 10 oder 11 für ihre Verwendung bei der Behandlung von Krebs.

13. Pharmazeutische Zusammensetzung, die dazu bestimmt ist, nach einem der Ansprüche 10 bis 12 verwendet zu werden, wobei der Krebs ein Karzinom, ein Tumor, eine Neoplasie, ein Lymphom, ein Melanom, ein Gliom, ein Sarcom oder ein Blastom ist.

14. Verfahren zur Herstellung der Kristallform I des Abexinostat Tosylats nach einem der Ansprüche 3 bis 9, wobei das Abexinostat in Anwesenheit von para-Toluolsulfonsäure in einem polaren Medium kristallisiert wird.

15. Verfahren zur Herstellung der Kristallform I des Abexinostat Tosylats nach Anspruch 14, wobei das polare Medium aus einem oder mehreren Lösungsmitteln, die aus Wasser, Alkoholen, Ketonen und Estern ausgewählt werden, gebildet wird.

16. Verfahren zur Herstellung der Kristallform I des Abexinostat Tosylats nach Anspruch 15, wobei das polare Medium ein binäres Gemisch ist, wovon einer der Bestandteile Wasser ist.

17. Verfahren zur Herstellung der Kristallform I des Abexinostat Tosylats nach Anspruch 16, wobei das polare Medium ein binäres Gemisch ist, das ausgewählt wird aus: Aceton/Wasser, Ethanol/Wasser, Isopropanol/Wasser und Methylethylketon/Wasser.

18. Verfahren zur Herstellung der Kristallform I des Abexinostat Tosylats nach einem der Ansprüche 14 bis 17, wobei die Kristallisation durch eine sehr kleine Menge der Kristallform I des Abexinostat Tosylats eingeleitet wird.

## Claims

1. *N*-hydroxy-4-{2-[3-(*N,N*-dimethylammomethyl)benzofuran-2-ylcarbonylamino]ethoxy}benzamide tosylate or one of its solvates.

2. Salt according to claim 1 of formula (II):

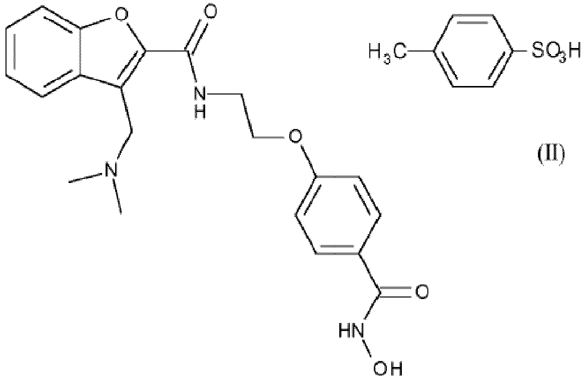

3. Crystalline form I of abexinostate tosylate according to one of claims 1 or 2, **characterised in that** it has an X-ray powder diffraction diagram having the following diffraction lines (Bragg's angle 2 theta, expressed in degrees ±0.2°): 6.50; 9.94; 11.35; 12.33; 14.08; 18.95; 21.08; 27.05.

4. Crystalline form I of abexinostate tosylate according to claim 3, **characterised in that** it has an X-ray powder diffraction diagram having the following diffraction lines (Bragg's angle 2 theta, expressed in degrees ±0.2°) : 6.50; 9.94; 11.35; 12.33; 14.08; 18.95; 19.61; 19.96; 21.08; 22.82; 23.61; 27.05.

5. Crystalline form I of abexinostate tosylate according to claim 3 or 4, **characterised in that** it has the following X-ray powder diffraction diagram measured on a PANalytical X'Pert Pro MPD diffractometer with an X'Celerator detector and expressed in terms of line position (Bragg's angle 2, expressed in degrees ±0.2°) and interreticular distance d (expressed in Å):
| **Line No.** | **Angle 2 theta (degrees)** | **Interreticular Distance (Å)** |
|---|---|---|
| 1 | 6.50 | 13.581 |
| 2 | 9.94 | 8.894 |
| 3 | 11.35 | 7.789 |
| 4 | 12.33 | 7.173 |
| 5 | 14.08 | 6.285 |
| 6 | 18.95 | 4.683 |
| 7 | 19.61 | 4.526 |
| 8 | 19.96 | 4.449 |
| 9 | 21.08 | 4.215 |
| 10 | 22.82 | 3.897 |
| 11 | 23.61 | 3.768 |
| 12 | 27.05 | 3.296 |

6. Crystalline form I of abexinostate tosylate according to one of claims 3 to 5, **characterised in that** it has a Raman spectrum having a significant peak at position 1608 cm⁻¹.

7. Crystalline form I of abexinostate tosylate according to one of claims 1 to 6, **characterised in that** it has a Raman spectrum having significant peaks at positions 940 cm⁻¹, 1088 cm⁻¹, 1132 cm⁻¹, 1242 cm⁻¹, 1360 cm⁻¹, 1608 cm⁻¹.

8. Crystalline form I of abexinostate tosylate according to one of claims 1 or 2, **characterised in that** it has a solid-state ¹³C CP/MAS NMR spectrum having the following peaks (expressed in ppm ± 0.2 ppm): 121.2 ppm, 122.1 ppm, 123.5 ppm 126.0 ppm, 126.8 ppm, 128.2 ppm, 128.9 ppm, 143.4 ppm, 144.6 ppm, 153.8 ppm, 159 ppm, 161.2 ppm and 162.1 ppm.

9. Crystalline form I of abexinostate tosylate according to claim 8, **characterised in that** it has a solid-state ¹³C CP/MAS NMR spectrum having the following peaks (expressed in ppm ± 0.2 ppm):
| **Peak No.** | **Chemical Shift (ppm)** | **Peak No.** | **Chemical Shift (ppm)** |
|---|---|---|---|
| 1 | 162.1 | 10 | 126.0 |
| 2 | 161.2 | 11 | 123.5 |
| 3 | 159.0 | 12 | 122.1 |
| 4 | 153.8 | 13 | 121.3 |
| 5 | 144.6 | 14 | 65.9 |
| 6 | 143.4 | 15 | 50.6 |
| 7 | 128.9 | 16 | 46.9 |
| 8 | 128.2 | 17 | 45.0 |
| 9 | 126.8 | 18 | 21.9 |

10. Pharmaceutical composition containing abexinostate tosylate according to one of claims 1 or 2 as active ingredient in association with one or more pharmaceutically acceptable excipients.

11. Pharmaceutical composition containing the crystalline form I of abexinostate tosylate according to one of claims 3 to 9 as active ingredient in association with one or more pharmaceutically acceptable excipients.

12. Pharmaceutical composition according to claim 10 or 11 for use in the treatment of cancer.

13. Pharmaceutical composition intended to be used according to one of claims 10 to 12, in which the cancer is a carcinoma, a tumour, a neoplasm, a lymphoma, a melanoma, a glioma, a sarcoma or a blastoma.

14. Process for preparing the crystalline form I of abexinostate tosylate according to one of claims 3 to 9, in which the abexinostate is crystallised in the presence of paratoluenesulphonic acid in a polar medium.

15. Process for preparing the crystalline form I of abexinostate tosylate according to claim 14, in which the polar medium is composed of one or more solvents selected from water, alcohols, ketones and esters.

16. Process for preparing the crystalline form I of abexinostate tosylate according to claim 15, in which the polar medium is a binary mixture, in which one of the constituents is water.

17. Process for preparing the crystalline form I of abexinostate tosylate according to claim 16, in which the polar medium is a binary mixture selected from: acetone/water, ethanol/water, isopropanol/water and methyl ethyl ketone/water.

18. Process for preparing the crystalline form I of abexinostate tosylate according to one of claims 14 to 17, in which the crystallisation is activated by a very small quantity of the crystalline form I of abexinostate tosylate.
